(19) Europäisches Patentamt
European Patent Office
Office européen des brevets

(11) EP 1 220 823 B1

(12) EUROPEAN PATENT SPECIFICATION

(45) Date of publication and mention of the grant of the patent:
**25.02.2004 Bulletin 2004/09**

(21) Application number: **00967799.8**

(22) Date of filing: **27.09.2000**

(51) Int Cl.[7]: **C07C 45/00**

(86) International application number:
**PCT/EP2000/009425**

(87) International publication number:
**WO 2001/023339 (05.04.2001 Gazette 2001/14)**

(54) **PROCESS FOR THE SYNTHESIS OF 2-HYDROXYPHENYL ALKYL KETONES**
VERFAHREN ZUR HERSTELLUNG VON 2-HYDROXYPHENYL ALKYL KETONEN
PROCEDE DE SYNTHESE DE 2-HYDROXYPHENYL ALKYL CETONES

(84) Designated Contracting States:
**AT BE CH CY DE DK ES FI FR GB GR IE IT LI LU MC NL PT SE**

(30) Priority: **30.09.1999 IT MI992038**

(43) Date of publication of application:
**10.07.2002 Bulletin 2002/28**

(73) Proprietor: **ENDURA S.p.A.**
**I-40121 Bologna (IT)**

(72) Inventors:
- **BORZATTA, Valerio**
  **I-40127 Bologna (IT)**
- **POLUZZI, Elisa**
  **I-40129 Bologna (IT)**
- **VACCARI, Angelo**
  **I-40127 Bologna (IT)**

(74) Representative: **Gervasi, Gemma, Dr.**
**Notarbartolo & Gervasi S.p.A.,**
**Corso di Porta Vittoria, 9**
**20122 Milano (IT)**

(56) References cited:
**US-A- 3 354 221**
**US-A- 4 652 683**
**US-A- 5 063 187**
**US-A- 5 525 323**

EP 1 220 823 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### FIELD OF THE INVENTION

The present invention belongs to the field of synthesis of phenyl ketones. A process is described for the synthesis of 2- hydroxyphenylalkyl ketones by catalyzed rearrangement starting from phenolic esters, characterized by the use of aluminosilicate-based catalysts having pre-determined chemical-physical characteristics.

### PRIOR ART

Hydroxyphenylalkyl ketones, in particular *ortho*-hydroxyacetophenone and *para*-hydroxyacetophenone (hereinafter referred to, respectively, as "2-HAP" and "4-HAP") are intermediate products useful in the synthesis of various products of industrial interest, such as catechol (US Ser. No. 661,552), guaiacol, acetylsalicylic acid (US. Ser. No. 633,832) and various other products in the field of essences, insecticides and pharmaceutical products *Adv. Catal.,* 1968, 18, 259.

Considering the industrial interest of these products, a great deal of work has been done in the field of their synthesis.

Hydroxyalkyl ketones are commonly obtained by means of Fries rearrangement on phenylacetates *(Appl. Catal.,* A: General 133, (1995), L1-L6). This reaction requires the use of Lewis acids or Brönsted acids, such as $AlCl_3$, as catalysts. However, these catalysts present the disadvantage of having to be used in high quantities and requiring a long process for recovery of the product; in addition, they cannot be recovered after use. Other catalysts used are $FeCl_3$, $TiCl_4$, or mineral acids, such as HF or $H_2SO_4$. All these catalysts produce a large quantity of organic by-products.

These drawbacks have been overcome by using zeolites as catalysts: these are used in smaller quantities and may be regenerated after use.

Zeolites are aluminosilicates the structure of which consists of tetrahedrons of $SiO_4$ and $AlO_4$ interconnected by atoms of oxygen to form a three-dimensional lattice. Various structures are obtained owing to the possibility of interconnecting the $SiO_4$ tetrahedrons and substituting the silicon atom with other elements, such as Al, B, Fe, and Ga.

The rearrangement of phenyl acetate, catalyzed by Zeolites, can be represented as follows:

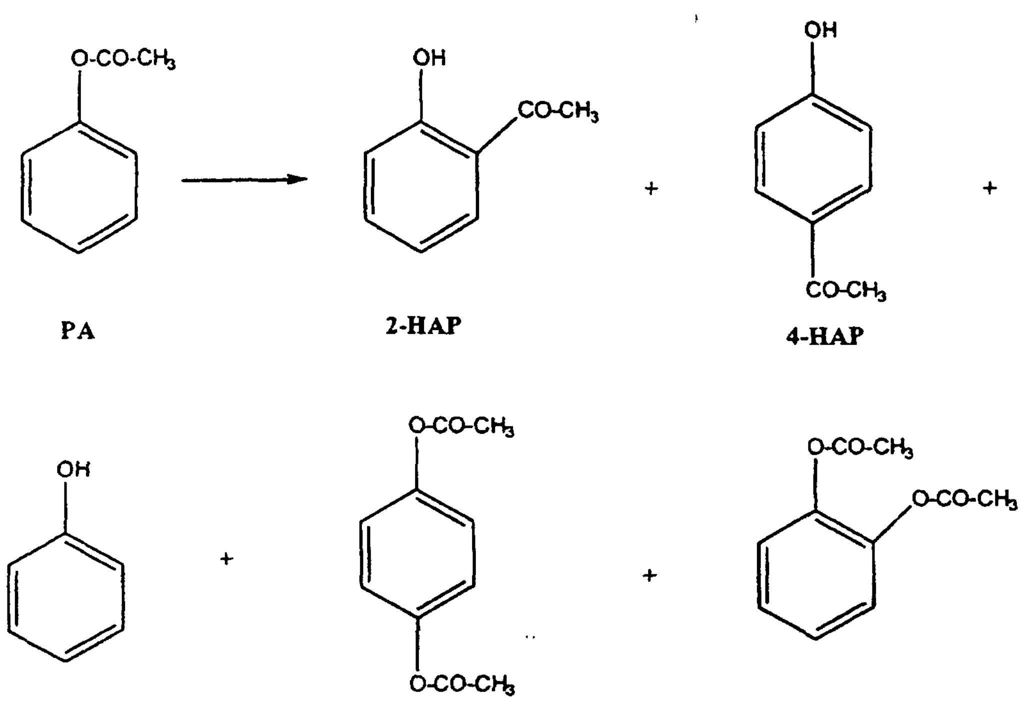

PA 2-HAP 4-HAP

PhOH 4-AcPA 2-AcPA

where: PA = phenyl acetate; 2-HAP = *ortho*-hydroxyacetophenone; 4-HAP = *para*-hydroxyacetophenone; PhOH = phenol; 4-AcPA = 4-acetylphenylacetate; and 2-AcPA = 2-acetylphenylacetate.

As is evident from the diagram, the rearrangement reaction leads to a mixture of 2-HAP, 4-HAP and a series of by-products, in particular phenol *(Adv. Catal.*, 1968, *op. cit.).* This reaction leads to a low yield of the desired isomer;

in addition, the rearrangement of the phenyl acetate (PA) is never quantitative, and part of this product does not react.

A great deal of research work has thus been directed at improving the efficiency of conversion of the phenyl acetate and its selective transformation into the desired isomer, whether 2-HAP or 4-HAP.

For instance, in US-A-4,668,826 zeolites of the type H-ZSM-5 are used in the direct acylation of phenol with acetic acid. The reaction is orthoselective, leading preferably to 2-HAP, but the percentage of conversion of the phenyl acetate does not exceed 40%. The same reaction is carried out in US-A-4,652,683 using silicalites containing predetermined amounts of alumina; the yield in 2-HAP does not improve, and high amounts of phenolic by-product are obtained. In *Tetr. Lett.,* 1989, 30(17), 2281-84, a reduction in the formation of phenol is achieved, using an anhydrous catalyst, at the cost, however, of a concomitant reduction in the general efficiency of reaction.

In *Tetr. Lett.,* 1993, 34(48), 7799-7800, an excellent orthoselectivity is obtained, using zeolites doped with metals, such as Ga, Ni, Pt, Pd; however, the efficiency of conversion remains on average around 65%, and the 2-HAP/phenol ratio is generally low (0.25-1.55).

In *Appl. Catal. A:* General, 123, 1995, 37-49, a comparison is made between the results of the Fries rearrangement reaction catalyzed by zeolites, conducted in discontinuous liquid phase or in continuous gaseous phase. The reaction proves more efficient in the liquid phase, and proceeds with a prevalent paraselectivity, leading to larger amounts of 4-HAP. In the gaseous phase, high amounts of phenol and other undesired by-products are obtained; the catalyst is moreover quickly inactivated, especially at low temperatures.

In a recent work *(Progr. Zeol. Microp. Mat.,* 1997, 105, 1197-1202), an evaluation was made of the effect of particular zeolites with large pores and high silica content (NCL-1) on the Fries rearrangement of the phenyl acetate: a modest orthoselectivity is obtained, as well as low efficiency of rearrangement and significant amounts of phenol.

Summarizing the teachings of the known art referred to above, it may be concluded that, although the use of zeolites has led to an improved industrial applicability of the Fries rearrangements, the syntheses in terms of hydroxyphenylalkyl ketones fail to meet completely the requirements of efficiency of rearrangement, selectivity of reaction, and reduction of formation of phenolic by-products. There thus remains the need for a process that leads efficiently and selectively to obtaining hydroxyphenylalkyl ketones, in particular 2-HAP and its derivatives.

## SUMMARY

It has now been surprisingly found that it is possible synthesize 2-hydroxyphenylalkyl ketones with high yields and high selectivity in a process of rearrangement catalyzed by specific crystalline aluminosilicates. These aluminosilicates have a silica/alumina ratio higher than 10, are made up of primary crystallites with a mean diameter of between 0.1 µm and 0.9 µm, and at least 20% of the primary crystallites are joined in agglomerates of dimensions between 5 µm and 500 µm; the primary crystallites and the agglomerates are bound together by means of finely ground alumina, obtained from hydrolysis of alumino-organic compounds. The surface area (determined by the BET method) of the catalyst is between 300 $m^2$/g and 600 $m^2$/g, and the volume of the pores (determined via porosity to mercury) is between 0.3 $cm^2$/g and 0.8 $cm^2$/g. The aforementioned crystalline aluminosilicates are present in the H form, and the finely ground alumina is contained in a weight percentage with respect to the weight of the catalyst of between 10wt% and 40 wt%.

The rearrangement catalyzed by these aluminosilicates involves a high conversion of the phenolic ester, a high orthoselectivity and a low formation of phenolic by-products, thus enabling the formation, in high yields, of 2-hydroxyphenylalkyl ketones, with particular application for the synthesis of 2-hydroxyacetophenone.

## DETAILED DESCRIPTION OF THE INVENTION

The subject of the present invention is a process for the synthesis of 2-hydroxyphenylalkyl ketones of formula (I)

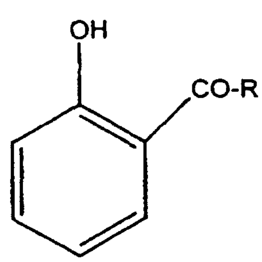

(I)

where R is a linear alkyl containing from 1 to 9 carbon atoms, preferably methyl, characterized by the interaction of a

compound of formula (II), where R has the aforementioned meanings,

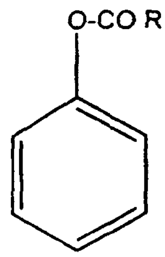

(II)

with a catalyst made up of crystalline aluminosilicates of the pentasyl type, where:

- said catalyst is made up of primary crystallites with a mean diameter of between 0.1 μm and 0.9 μm;
- at least 20% of the crystallites are joined in agglomerates of dimensions between 5 μm and 500 μm;
- the primary crystallites or the agglomerates are bound together by means of finely ground alumina obtained from the hydrolysis of alumino-organic compounds, the said alumina being contained in a weight percentage with respect to the weight of the catalyst of between 10 wt% and 40 wt%;
- the surface area (determined with the BET method) of the catalyst is between 300 $m^2/g$ and 600 $m^2/g$ and the volume of the pores (determined via porosity to mercury) is between 0.3 $cm^2/g$ and 0.8 $cm^2/g$;
- the alluminosilicate is present in the H form;
- the silica/alumina ratio is higher than 10.

The BET method of determination of the surface area is a widely known standard method (see, for example, *J. Am.* Soc., 60, 1938, p.309).

These aluminosilicates, already described in the patent application EP-A-369364, are characterized by a particular porosity that is established when the primary crystallites aggregate to form agglomerates with the addition of the aluminous binder. The patent EP-A-369364 describes the use of these compounds in the conversion of olefins into diesel, in the oil industry sector.

It has now been unexpectedly found, and this forms the subject of the present invention, that these compounds may to be used as catalysts in the Fries rearrangement of phenyl acetate, leading to obtaining 2-hydroxyphenylalkyl ketones, in particular *ortho*-hydroxyacetophenone (2-HAP), in conditions of efficiency and selectivity not as yet attained by the prior art.

The aluminosilicates that may be used in the present invention may be obtained with the following methodology:

a) production of a gel in an aqueous environment containing a source of silicon, a source of aluminium, a source of alkali and a templating agent, such as tetrapropylammonium hydroxide. From the mixture, treated at high pressures and temperatures, there are formed, in a controlled manner, primary crystallites of alluminosilicate with diameters of between 0.1 μm and 0.9 μm;
b) pre-aggregation of the primary crystallites by addition of flocculants, such as copolymers of acrylamide and derivatives of acrylic acid, and separation of the pre-aggregates obtained;
c) ion exchange in an aqueous environment of the pre-aggregates with a substance capable of donating protons when heated, separation and immediate calcining of the product obtained, and isolation of the fraction of agglomerates of dimensions between 5 μm and 500 μm;
d) mixing of the agglomerates with finely ground hydrated alumina, said alumina being preferably in a peptizable form; and
e) final calcining of the product obtained.

For greater details of description and preparation of these aluminosilicates, the reader is referred to the patent application EP-A-369364, which is here incorporated by reference in its entirety.

Preferred examples of such products are distributed commercially, by Süd-Chemie under the names T-4480 powder, T-4480 Extr. 1/16", and Ex-1720 Pt-1. A further subject of the present invention is therefore a process for the synthesis of 2-hydroxyphenylalkyl ketones of formula (I)

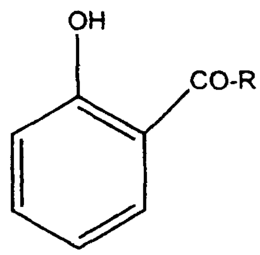

(I)

where R is a linear alkyl containing from 1 to 9 carbon atoms, preferably methyl, characterized by the interaction of a compound of formula (II), where R has the aforementioned meanings,

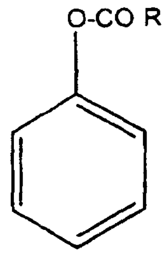

(II)

with a catalyst chosen from among T-4480 powder, T-4480 Extr. 1/16", and Ex-1720 Pt-1.

The rearrangement reaction that leads to obtaining the compounds of formula (I) is preferably conducted in conditions of gas flow in appropriate reactors, for instance, of a tubular structure. These plants are characterized by a feed zone, a reaction zone and a zone for collection of the products of reaction. The compound of formula II (for instance, PA) is vehicled by a vector gas, and the resultant flow is set in contact, for a predetermined period of time, with the aluminosilicates having the chemical-physical characteristics defined above.

The vector gas utilized is an inert gas, for instance nitrogen, helium, or argon. In reactions on zeolites in the gaseous phase it is common practice to add a small amount of water to increase the lifetime of the catalyst; in the present process, in order to obtain high yields of 2-HAP, it is preferable for the gas to contain not more than 1% of water.

The catalyst is set inside the reactor in such a form that it enables convenient and efficient contact with the gas flow, for instance by forming a bed of catalyst through which the gas flows. Before carrying out the reaction, it is preferable to activate the catalyst by means of pre-heating under flow of inert gas.

When the conditions of activation are attained, the phenolic ester of formula (II) is added to the flow of vector gas. The phenolic ester is incorporated in the vector gas in volume proportions generally of between 1% and 20%, preferably between 5% and 15%, or more preferably 10% with respect to the total gas (gas + phenolic ester). These percentages are to be understood as being measured at ambient pressure and at the reaction temperature. Preferably, the phenolic ester is added to the vector gas by means of a infusion pump in a line heated at a temperature higher than the boiling temperature of the ester. In the case of PA the normal operating temperature is 200°C.

The spatial velocity (WHSV) is normally between 0.1 $h^{-1}$ and 1.0 $h^{-1}$, preferably between 0.25 $h^{-1}$ and 0.75 $h^{-1}$, and more preferably is 0.5 $h^{-1}$.

By spatial velocity is meant the ratio between the rate of feed [grams of (gas+reagent) per hour] and the quantity [in grams] of catalyst, according to the formula:

$$WHSV\ (h^{-1}) = R_{feed}\ (g/h)\ /\ Q_{cat}\ (g)$$

The reaction temperature is between 220°C and 280°C, preferably between 240°C and 265°C, or more preferably is 256°C. By the term temperature of reaction" is meant the temperature that characterizes the reaction zone of the reactor.

For a general description of the technology of reactions in gas flow, the reader is referred to the text "Chemical Reaction Engineering" published by John Wiley & Sons, New York, 2nd ed., 1972.

The present invention is now described by means of the following non-limiting experimental examples.

## EXPERIMENTAL PART

1. Catalysts tested

The performance of various acidic catalysts in the rearrangement of PA to form 2-HAP were analyzed. Particular aspects studied were the orthoselectivity of the reaction, the production of by-products, and the total yields of 2-HAP.

As reference catalysts, various types of zeolites were used, ones commonly used in the catalysis of reactions in gaseous phase, in acidic form or partially exchanged with Ga or La. The $SiO_2/Al_2O_3$ ratio of the tested zeolites ranged between 25 and 120. More specific characteristics of these products are illustrated in the table below.

Table 1.

| Specimen | $SiO_2/Al_2O_3$ | Impregnat. Element | Channel dimension (Å) |
|---|---|---|---|
| H-ZSM-5 | 28 | - | 5.3*5.6,5.5*5.1 |
| H-ZSM-5 | 120 | - | 5.3*5.6,5.5*5.1 |
| H-Y | 30 | - | 24.28 |
| H-β | 25 | - | n.d. |
| H-ZSM-5 | 28 | Ga | - |
| H-ZSM-5 | 28 | La | - |
| H-Y | 30 | Ga | - |
| H-β | 25 | Ga | - |

The performance of these catalysts was compared to that of the catalysts T-4480 powder, T-4480 Extr. 1/16" and Ex-1720 Pt-1 belonging to the family of compounds described in the patent EP-A-369364.

**2.** Laboratory microplant

The catalytic tests were conducted in conditions of gas flow using a laboratory microplant. The plant may be divided into three main zones: (a) feed zone, (b) reaction zone, (c) zone of collection of products.

A. **FEED ZONE**

The flow of He is regulated by a mass flowmeter calibrated with a bubble flowmeter. The phenylacetate (PA) is supplied by an infusion pump and is introduced into the line heated at 200°C and controlled by an iron/Constantan thermocouple.

B. **REACTION ZONE**

The tubular reactor consists of a stainless steel tube with a section of 1 inch and a length of approximately 50 cm. Inside the tube, a perforated diaphragm blocks the catalyst inside the reactor, enabling the passage of the flow of gas. In contact with the tube are two electrically heated copper jaws controlled by means of a thermocouple. The ensemble is insulated by perlite and enclosed in a brass cylinder.

Inserted in the catalytic bed is a 1/8-inch stainless steel tube inside which a mobile thermocouple can slide, thus enabling measurement of the reaction temperature at different heights of the catalytic bed. The gases coming out are maintained at 100°C by means of a resistor controlled by a thermoregulator to prevent crystallization of the heavier products.

C. **ZONE FOR COLLECTION OF PRODUCTS**

At output from the hot zone is set a glass crystallizer at room temperature that enables collection of the condensates. Subsequently, there are two bubble adsorbers containing acetone and set in ice (for the collection of the products that do not condense in the crystallizer) and a bubble adsorber containing absolute ethanol to transform the ketene that may possibly be formed in acetyl acetate.

3. Conditions of reaction

The experimental conditions are the ones that yielded the best results following upon a prior screening. These conditions were:

- Temperature of the incoming band = 200°C.
- Temperature at output from the reactor = 100°C.
- Reaction temperature (i.e., the temperature inside the reactor in the reaction zone) = 256°C.
- Temperature of the catalytic bed = 265°C.
- Activation of the catalyst: 5 hours at 300°C in He (60 ml/min).
- Feed:

    quantity of PA contained in the inert gas =10%.
    spatial velocity (WHSV) inside the reactor = 0.5 $h^{-1}$.

- Pressure: atmospheric

4. Results

After 4 hours from the start of reaction, the reaction products were determined quantitatively via gas-chromatography analysis. The analyses yielded the following results:

Table 2

| | Catalyst | PA Conv. | 2-HAP yield | 2-HAP/4-HAP | 2-HAP/PhOH |
|---|---|---|---|---|---|
| 1 | **H-ZSM-5(28)** | 72.8 | 22.0 | 42.1 | 0.30 |
| 2 | **H-ZSM-5(120)** | 52.1 | 7.3 | 2.9 | 0.27 |
| 3 | **Ga-ZSM-5(28)** | 33.3 | 17.4 | 3.3 | 0.42 |
| 4 | **La-ZSM-5(28)** | 78.6 | 4.8 | 163.3 | 0.2 |
| 5 | **HY-30** | 66.8 | 16.6 | * | 0.3 |
| 6 | **Ga-Y** | 85.9 | 3.5 | 17.5 | 0.33 |
| 7 | **H-β (25)** | 75.4 | 21.2 | * | 0.42 |
| 8 | **T-480Extr.1/16"** | 74.5 | 32.9 | 17.3 | 1.5 |
| 9 | **T-4480 powder** | 62.0 | 73.6 | 26.6 | 2.5 |
| 10 | **Ex-1720 Pt-1** | 68.1 | 59.4 | 19.8 | 2.1 |

* = only 2-HAP was found

From the data given in Table 2 it may be noted that the traditional zeolites (specimens 1, 2 and 5-7) have a high 2-HAP / 4-HAP selectivity, but the yield in 2-HAP is quite modest in that a substantial proportion of PA is converted into phenol, as is evident from the low values of the 2-HAP/PhOH ratio.

The doping of the zeolites with metals (see specimens 3, 4 and 6) does not improve the yield in 2-HAP, nor does it reduce the formation of the PhOH by-product.

Instead, the catalysts according to the present invention (see specimens 8-10) revealed a high 2-HAP/4-HAP selectivity, together with a value of 2-HAP/PhOH ratio considerably higher than that obtained for any of the reference specimens.

As emerges from the Table, the synergy between these two factors leads to 2-HAP yields that are surprisingly higher than those obtained from all the reference specimens.

The yields in further by-products of reaction (2-AcPA, 4-AcPA) are lower than 0.2%.

Further tests were carried out on a longer production cycle (4 hours/day for three days) to verify the persistence of activity and the constancy of the yields over time.

During the entire duration of the test, none of the above-mentioned reference catalysts managed to exceed or even approach the yield values found in the process forming the subject of the invention.

The persistence of high yield values over time for the process forming the subject of the invention enables retardation of the cycles of reactivation of the catalyst, thus increasing the productivity of the plants.

## Claims

**1.** A process for the synthesis of 2-hydroxyphenylalkyl ketones of formula (I)

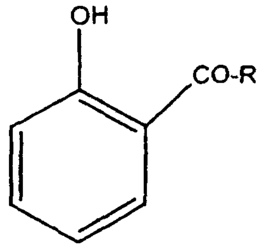

(I)

where R is a linear alkyl containing from 1 to 9 carbon atoms, **characterized by** the interaction of a compound of formula (II), where R has the meanings indicated above

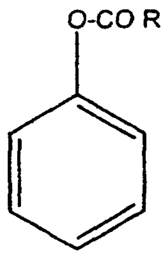

(II)

with a catalyst made up of crystalline aluminosilicates of the pentasyl type, where

- said catalyst is made up of primary crystallites with a mean diameter of between 0.1 µm and 0.9 µm;
- at least 20% of the crystallites are joined in agglomerates of dimensions of between 5 µm and 500 µm;
- the primary crystallites or the agglomerates are bound together by means of finely ground alumina obtained from the hydrolysis of alumino-organic compounds, the said alumina being contained in a weight percentage with respect to the weight of the catalyst of between 10 wt% and 40 wt%;
- the surface area (determined using the BET method) of the catalyst is between 300 $m^2$/g and 600 $m^2$/g and the volume of the pores (determined via porosity to mercury) is between 0.3 $cm^2$/g and 0.8 $cm^2$/g;
- the alluminosilicate is present in the H form; and
- the silica/alumina ratio is higher than 10.

**2.** A process according to Claim 1, where R is chosen between methyl and ethyl.

**3.** A process according to Claims 1 and 2, where said catalyst is chosen between the products T-4480 powder, T-4480 Extr. 1/16", and Ex-1720 Pt-1, (Süd-Chemie).

**4.** A process according to Claims 1-3, conducted in the gaseous phase.

**5.** A process according to Claim 4, conducted in the following conditions:

- temperature of reaction of between 220°C and 280°C;
- spatial velocity of between 0.1 $h^{-1}$ and 1.0 $h^{-1}$; and
- concentration of compound of formula (II) in the gaseous phase of between 1 vol% and 20 vol% with respect to the total gas (i.e., gas + compound of formula II).

**6.** A process according to Claim 4, conducted in the following conditions:

- temperature of reaction of between 240°C and 265°C;
- spatial velocity of between 0.25 $h^{-1}$ and 0.75 $h^{-1}$; and
- concentration of compound of formula (II) in the gaseous phase of between 5% and 15%, by volume

**7.** A process according to Claim 4, conducted in the following conditions:

- temperature of reaction: 256°C;
- spatial velocity: 0.5 $h^{-1}$; and
- concentration of compound of formula (II) in the gaseous phase: 10 vol%.

**8.** A process according to Claims 4-7, where the gas utilized to produce the gaseous phase is chosen from among nitrogen, helium, argon or their mixtures.

**9.** A process according to Claims 4-8, where the gas utilized to produce the gaseous phase contains at most 1% of water.

**10.** A process according to Claims 4-9, where the catalyst is activated by means of pre-heating under flow of inert gas before the reaction is carried out.

**Patentansprüche**

**1.** Verfahren zur Herstellung von 2-Hydroxyphenylalkylketonen mit der Formel (I)

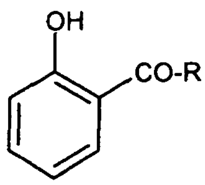

(I)

wobei R eine lineare Alkylgruppe mit 1 bis 9 Kohlenstoffatomen ist, **dadurch gekennzeichnet, dass** eine Verbindung mit der Formel (II),

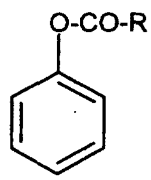

(II)

worin R die oben genannten Bedeutungen hat, mit einem Katalysator aus kristallinen Alumosilicaten vom Pentasil-Typ wechselwirkt, wobei

- der genannte Katalysator aus primären Kristalliten mit einem mittleren Durchmesser zwischen 0,1 µm und 0,9 µm besteht;
- mindestens 20 % der Kristallite in Agglomeraten in Größen zwischen 5 µm und 500 µm vereinigt sind;
- die primären Kristallite oder Agglomerate mittels fein gemahlenem Aluminiumoxid zusammengehalten werden, welches durch die Hydrolyse von aluminoorganischen Verbindungen erhalten wurde, wobei genanntes Aluminiumoxid in Gewichtsanteilen, bezogen auf das Gewicht des Katalysators, zwischen 10 Gew.-% und 40 Gew.-% enthalten ist;
- die Oberfläche (bestimmt mittels des BET-Verfahrens) des Katalysators zwischen 300 $m^2/g$ und 600 $m^2/g$ und das Porenvolumen (bestimmt mittels der Porosität gegenüber Quecksilber) zwischen 0,3 $cm^2/g$ und 0,8 $cm^2/g$ liegt;
- das Alumosilicat in der H-Form vorliegt; und
- das Silicium/Aluminiumoxid-Verhältnis größer als 10 ist.

2. Verfahren gemäß Anspruch 1, wobei R Methyl -oder Ethyl- ist.

3. Verfahren gemäß den Ansprüchen 1 und 2, wobei der genannte Katalysator ausgewählt wird aus den Produkten T-4480-Pulver, T-4480 Extr. 1/16", und EX-1720 Pt-1 (Süd-Chemie).

4. Verfahren gemäß den Ansprüchen 1 bis 3, welches in der Gasphase ausgeführt wird.

5. Verfahren gemäß Anspruch 4, ausgeführt unter den folgenden Bedingungen:

   . Reaktionstemperatur zwischen 220 °C und 280 °C;

   . Raumgeschwindigkeit zwischen 0,1 $h^{-1}$ und 1,0 $h^{-1}$; und

   . Konzentration der Verbindung mit der Formel (II) in der Gasphase zwischen 1 Vol.-% und 20 Vol.-% bezogen auf das gesamte Gas (d.i. Gas + Verbindung mit der Formel II).

6. Verfahren gemäß Anspruch 4, ausgeführt unter den folgenden Bedingungen:

   . Reaktionstemperatur zwischen 240 °C und 265 °C;

   . Raumgeschwindigkeit zwischen 0,25 $h^{-1}$ und 0,75 $h^{-1}$; und

   . Konzentration der Verbindung mit der Formel (II) in der Gasphase zwischen 5 Vol.-% und 15 Vol.-%.

7. Verfahren gemäß Anspruch 4, ausgerührt unter den folgenden Bedingungen:

   . Reaktionstemperatur: 256 °C;

   . Raumgeschwindigkeit: 0,5 $h^{-1}$; und

   . Konzentration der Verbindung mit der Formel (II) in der Gasphase: 10 Vol.-%.

8. Verfahren gemäß den Ansprüchen 4 bis 7, wobei das zur Erzeugung der Gasphase verwendete Gas ausgewählt wird aus Stickstoff, Helium, Argon oder ihren Mischungen.

9. Verfahren gemäß den Ansprüchen 4 bis 8, wobei das zur Erzeugung der Gasphase verwendete Gas höchstens 1 % Wasser enthält.

10. Verfahren gemäß den Ansprüchen 4 bis 9, wobei der Katalysator mittels Vorheizen unter einem Inertgasstrom vor der Ausführung der Reaktion aktiviert wird.

**Revendications**

1. Procédé pour la synthèse de 2-hydroxyphénylalkyl cétones de formule (I)

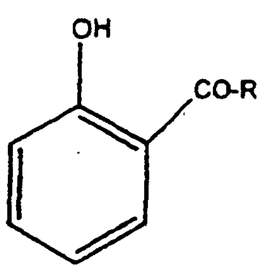

(I)

où R est un alkyle linéaire contenant de 1 à 9 atomes de carbone, **caractérisé par** l'interaction d'un composé de formule (II), où R a les significations indiquées ci-dessus

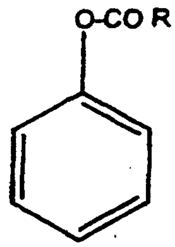

(II)

avec un catalyseur formé d'aluminosilicates cristallins du type pentasyle, où

- ledit catalyseur est formé de cristallites primaires avec un diamètre moyen compris entre 0,1 μm et 0,9 μm;
- au moins 20% des cristallites sont joints en agglomérats de dimensions entre 5 μm et 500 μm;
- les cristallites primaires ou les agglomérats sont liés ensemble au moyen d'alumine finement broyée obtenue de l'hydrolyse de composé alumino-organique, ladite alumine étant contenue à un pourcentage pondéral par rapport au poids du catalyseur entre 10% en poids et 40% en poids.
- l'aire superficielle (déterminée en utilisant la méthode BET) du catalyseur est entre 300 $m^2$/g et 600 $m^2$/g et le volume des pores (déterminé via porosité au mercure) est entre 0,3 $cm^2$/g et 0,8 $cm^2$/g.
- l'aluminosilicate est présent sous la forme H; et
- le rapport silice/alumine est supérieur à 10.

**2.** Procédé selon la revendication 1, où R est choisi entre méthyle et éthyle.

**3.** Procédé selon les revendications 1 et 2, où ledit catalyseur est choisi entre les produits poudre T-4480, T-4480 Extr. 1/16", et Ex-1720 Pt-1, (Süd-Chemie).

**4.** Procédé selon les revendications 1-3, entrepris en phase gazeuse.

**5.** Procédé selon la revendication 4, entrepris dans les conditions suivantes:

- température de réaction entre 220°C et 280°C;
- vitesse spatiale entre 0,1 $h^{-1}$ et 1,0 $h^{-1}$; et
- concentration du composé de formule (II) dans la phase gazeuse entre 1% en volume et 20% en volume par rapport au gaz total (i.e., gaz + composé de formule II).

**6.** Procédé selon la revendication 4, entrepris dans les conditions suivantes:

- température de la réaction entre 240°C et 265°C;
- vitesse spatiale entre 0,25 $h^{-1}$ et 0,75 $h^{-1}$; et
- concentration du composé de formule (II) dans la phase gazeuse entre 5% et 15% en volume.

**7.** Procédé selon la revendication 4, entrepris dans les conditions suivantes:

- température de réaction 256°C;
- vitesse spatiale 0,5 $h^{-1}$; et
- concentration du composé de formule (II) dans la phase gazeuse 10% en volume.

**8.** Procédé selon les revendications 4-7, où le gaz utilisé pour produire la phase gazeuse est choisi parmi azote, hélium, argon ou leurs mélanges.

**9.** Procédé selon les revendications 4-8, où le gaz utilisé pour produire la phase gazeuse contient au plus 1% d'eau.

**10.** Procédé selon les revendications 4-9, où le catalyseur est activé par préchauffage sous un écoulement de gaz inerte avant que la réaction ne soit effectuée.